# EUROPEAN PATENT SPECIFICATION

(11) **EP 2 143 386 B1**
(45) Date of publication and mention of the grant of the patent: **15.05.2013**
(21) Application number: 09251776.2
(22) Date of filing: 10.07.2009
(51) Int. Cl.: A61B 10/00, B65D 81/26

(54) **Bag assembly**
Beutelanordnung
Ensemble de sac

(30) Priority: 10.07.2008 GB 0812601
(43) Date of publication of application: 13.01.2010
(73) Proprietor: Jones & Brooks Ltd, Rochdale, OL16 2SH (GB)
(72) Inventor: Blair, Ronald Victor, Littleborough OL15 0DD (GB)
(74) Representative: Atkinson, Peter Birch

(56) References cited:
- WO-A-85/01272
- WO-A-93/04946
- GB-A- 2 400 361
- US-A- 5 833 058
- US-A1- 2006 233 467

## Description

The present invention relates to a bag assembly of the type intended for use in transferring a container holding a specimen of a liquid body fluid (e.g. blood, urine or sputum), optionally together with written information relating to that sample, to a laboratory where the sample may be analysed. The sample may, for example, be one collected in a hospital, a Doctor's surgery or in a patient's own home.

Such bag assemblies are known and comprise a backing component (usually of paper) to which is attached a plastics bag into which the container (holding the liquid sample) is placed. It is generally the case that the bag assembly is such that, once the container has been placed in the plastics bag, a flap or the like may be sealed over the mouth of the bag to retain the container therein until such time as the bag is opened and the container removed from analysis of the sample. Examples of such bag assemblies are disclosed, for example, in GB-A-2 221 208 and GB-A-2 400 361 (both in the name of Jones & Brooks Ltd).

Such bag assemblies are generally produced by laying on to an advancing web of a plastics material (which ultimately forms the plastics bag of the assembly) on to an advancing web of the backing component. The plastics web has a longitudinal fold so as, in effect, to comprise two layers. Subsequently the advancing web is passed through a heat seal arrangement at which successive (longitudinally spaced) transverse heat seals are made to join the two layers of the plastics web together along the heat seals. Subsequently the plastics web and the backing component are severed along the lines of the heat seals. There is thus produced a bag assembly in which the plastics bag has an open mouth, two sides closed by the heat seals, and a third side closed by the aforementioned fold.

Given that the bag assembly is intended to hold a liquid sample (e.g. blood, urine or sputum) there is always the potential risk that the liquid sample may inadvertently spill into the plastics bag. This can arise, for example, because a top on the container has not been properly applied. Alternatively the container itself may be damaged in transit. Thus a person opening the plastics bag to gain access to the sample may be a risk of exposure to spilt liquid in the bag. For this reason, legislation requires that the plastics bag incorporates a pad of an absorbent material capable of absorbing 40ml of liquid In the event of spillage from the container Into the bag. Such pads of absorbent material may be incorporated in the plastics bag. However this requires that the pads be individually Inserted Into the pre-formed bags (e.g. by hand) and this is a time-consuming operation.

WO/93/04946 discloses a bag assembly having a single sheet backing component to which is affixed a plastics bag for receiving and holding a container of a liquid sample. The bag is formed from two sheets of liquid impervious plastics material bonded together to form the bag. Within the bag is a pad of absorbent material bonded to an inner surface of the bag. Part of the backing component provides a fold-over flap for closure of the bag.

US 2006/233467 discloses pre-padded bags in which an absorbent pad may be provided.

It is therefore an object of the present invention to obviate or mitigate the abovementioned disadvantages.

According to a first aspect of the present invention there is provided a bag assembly comprising a backing component and affixed thereto a plastics bag for receiving and holding a container of a liquid sample, the plastics bag being formed from two sheets of liquid impervious plastics material bonded together to form the bag and to leave a mouth therefor and the bag containing a pad of absorbent material characterised In that the backing component composes two layers bonded together to form a pouch, one of said layers forming the backing component extends beyond the mouth of the pouch to form a flap for the backing component, one of the sheets of plastics material forming the bag extends beyond the mouth of the bag to form a plastics flap for the bag, and the reverse of the plastics flap is bonded to the front of the backing component flap with the plastics bag overlying the mouth of the pouch.

According to a second aspect of the present invention there is provided a method of producing a bag assembly, the method comprising the steps of:
(a) advancing a web of a backing component,
(b) laying onto said advancing web of the backing component an advancing first web of a liquid Impervious plastics material and bonding said webs together along a join line,
(c) depositing pads of absorbent material at Intervals onto the exposed surface of the advancing first plastics web,
(d) laying an advancing second web of liquid impervious plastics material onto the first web of plastics material so as to sandwich the absorbent pads between the first and second plastics webs,
(e) bonding said first and second plastics web together along a line extending lengthwise of the plastics webs and along first lines extending transversely of the plastics webs between successive absorbent pads, and
(f) severing the web produced in step (e) along said first transverse lines, wherein the backing component comprises first and second webs bonded together along one lengthwise glue line and along spaced second transverse glue lines to form successive pockets in the backing component, wherein, in step (e), the first transverse lines are formed coincident with said second transverse lines and wherein, in step (f), the web is severed along the first and second transverse lines, wherein one longitudinal edge of the first web of the backing component is coincident with one longitudinal edge of the second web of the backing component and said coincident longitudinal lines are bonded together along said lengthwise line, wherein the first web of the backing component is wider than the second web thereof whereby said first web has a portion overlapping the second web and wherein the first web of plastics material is, in step (b) affixed along said join line to the overlapping portion on the same face thereof as the second web of the backing component.

Therefore, in accordance with the invention, the plastics bag is formed from separate sheets of plastics material. This enables the bag assembly to be produced by a continuous process as defined for the second aspect of the invention. More particularly, in such a process, a travelling web assembly comprised of a backing component web and a first web of a liquid impervious plastics material may be formed. Absorbent pads may be deposited in spaced locations successively onto the first web of plastics material subsequently a second liquid impervious plastics web may be laid on to the first web, thereby sandwiching the absorbent pads between the two plastics webs which may then be heat sealed together as defined in step (b). Subsequently the web may be severed as defined I step (f) to produce the bag assemblies.

In a preferred embodiment of the bag assembly in accordance with the first aspect of the invention, the absorbent pad is bonded to an inner surface of the plastics bag. In the production process, this may be achieved either by applying adhesive to the plastics web and/or to the absorbent pad before it is laid on to the first web of plastics material.

The pad is preferably of a material that is capable of absorbing 40ml of liquid.

The invention will now be illustrated, by way of example only, with reference to the accompanying drawings, in which:
Fig 1 illustrates one embodiment of bag assembly in accordance with the invention;
Fig 2 is an exploded perspective view of the bag assembly illustrated in Fig 1;
Fig 3 schematically illustrates an apparatus for use in producing the bag assembly illustrated in Fig 1; and
Fig 4 illustrates an intermediate stage in the production of the bag assembly shown in Fig 1.

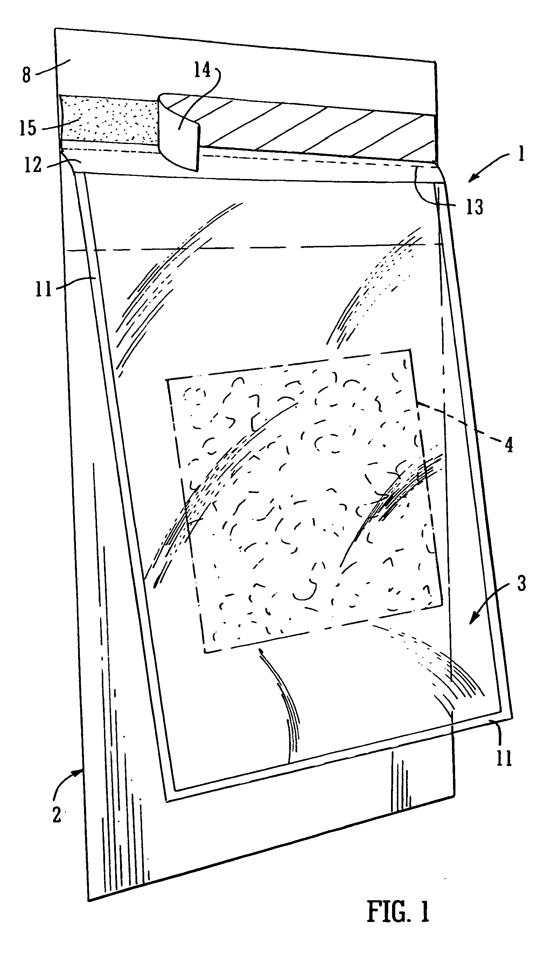

Referring to Fig 1 there is illustrated a bag assembly 1 which is intended for use in transferring a container holding a body fluid (e.g. blood, urine or sputum) together with written information relating to that sample to a laboratory where the sample may be analysed. The sample may, for example, be one collected in a hospital or in a patient's own home.

The bag assembly 1 is, in effect, comprised of two principal parts, namely a paper pouch assembly 2 (for holding the aforementioned written information) and a plastics bag assembly 3 (for holding the aforementioned container of the body sample). Provided within the bag assembly 3 is a pad of absorbent material 4 which is capable of absorbing 40 ml of liquid within two seconds in the event that there is a leak from the container. Although not illustrated in Fig 1, the front and rear faces of pouch assembly 2 may be provided with printed information (e.g. instructions for use of the bag assembly 1). Additionally plastics bag assembly 3 may also be printed with information, e.g. a hazard warning.

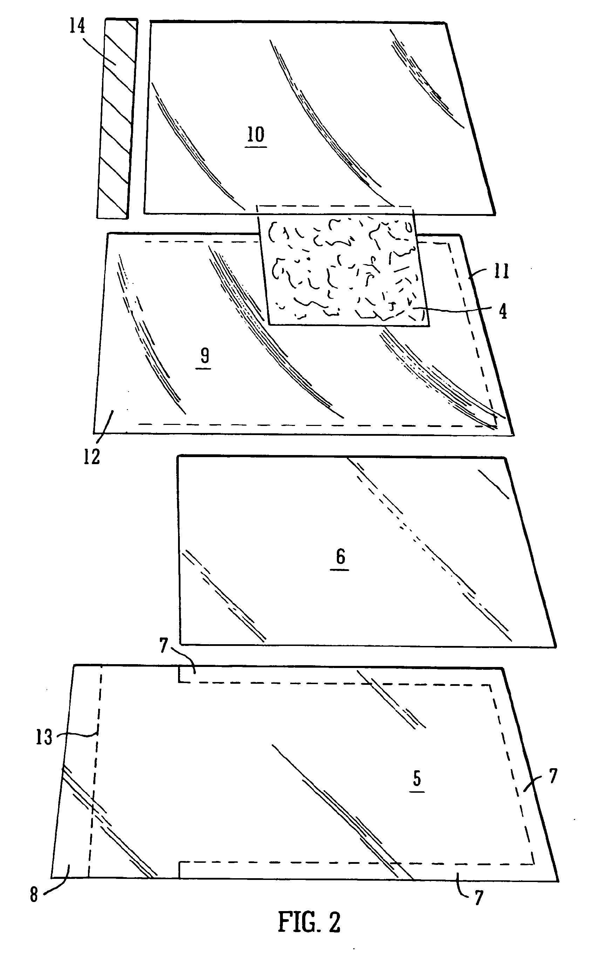

Paper pouch assembly 2 is formed of two rectangular paper sheets 5 and 6 (see also Fig 2) which are of the same width but with the former having a length X greater than the length Y of the latter. As will be appreciated from Fig 1, the pouch assembly 2 is formed by virtue of paper sheet 6 being superimposed on paper sheet 5 so that their right hand (as viewed in Fig 2) width wise edge and two length wise edges are coincident, the paper sheets 5 and 6 being bonded together along these coincident edges (referenced as 7) to form the pouch, leaving a portion of the sheet 5 as a flap 8 extending leftward (as viewed in Fig 2) beyond the mouth of the pouch. The plastics bag assembly 3 is formed of two plastics (e.g. polyethylene) sheets 9 and 10 between which is sandwiched the absorbent pad 4. Sheets 9 and 10 are of equal width but sheet 9 is of a length greater than sheet 10. In the bag assembly 3, the upper plastic sheet 10 is positioned so that one of its width wise edges and two of its length wise edges are coincident with corresponding edges of lower sheet 9, these coincident edges (referenced as 11) being heat-sealed together to form a bag with there being a plastics flap 12 extending beyond the mouth of the bag.

As seen in Fig 1, a line of perforations 13 is formed in the paper flap 8 so as to extend the whole way across the width of sheet 5. The free edge of plastics flap 12 is adhesively bonded (along its reverse face) to the paper flap 8 by a line of adhesive provided above (as viewed in Fig 1) the line of perforations 13.

Additionally, a removable paper strip 14 is bonded to the front side of the free edge of plastics flap 12 by virtue of a line of adhesive 15 on the front face of the plastics flap 12.

In order to ensure that it is located securely in position the absorbent pad 4 (provided within the plastics bag) is adhesively bonded to the plastics sheet 9.

It will be appreciated that the arrangement is such that the plastics bag may be lifted clear of the mouth of the paper pouch assembly 2 (whilst, of course, still being adhesively bonded to the paper flap 8) to allow written information to be inserted into the paper pouch.

In use of the bag assembly 1, a container (e.g. a screw-top container) of the body fluid sample that is to be analysed is located in the plastics bag 3. Written information relating to the sample (e.g. details of the analysis tests required) may be inserted in the paper pouch 2.

The plastics bag 2 is now sealed by removing the paper strip 14 and folding the paper flap 8 along the perforation line 13 so that the adhesive 15 along the front facial free edge of plastics flap 12 bonds that edge of the flap to the front face of sheet 10.

In the normal course of events, the container (holding the liquid sample) contained within the plastics bag will remain securely sealed until the bag is opened and the container is removed. However in the event of inadvertent leakage of liquid sample from the container (e.g. because the top has not been properly applied) free liquid within the plastics bag is absorbed by the absorbent pad 5, thereby minimising any risk to personnel who are required to open the plastics bag and remove the container for the purpose of analysing the sample.

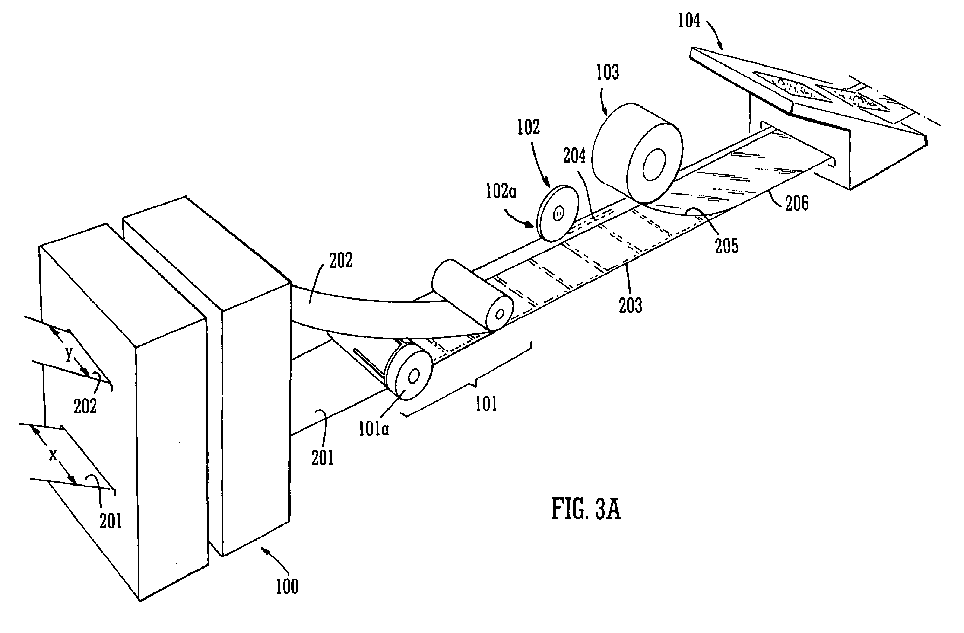

In the next stage of the process, a glue line 204 is applied parallel to the web 201 on a portion of the upper surface thereof not overlaid by web 202. This glue line 304 is depicted schematically in Fig 3A.

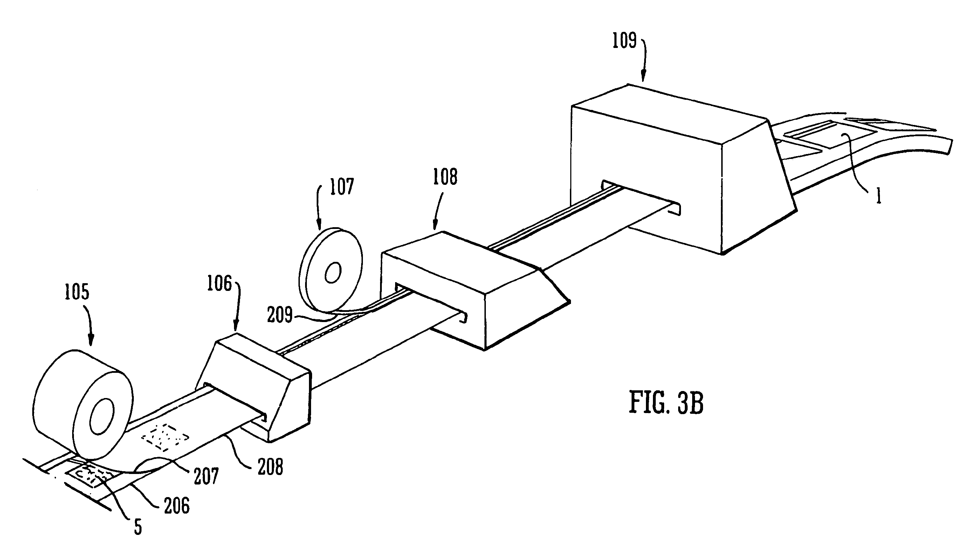

Reference is now made to Fig 3 which schematically illustrates apparatus for use in the production of the bag assembly 1 described above.

As shown in Fig 3, the illustrated apparatus comprises the following stations going in succession from the upstream end to the downstream end of the apparatus:
paper web printing station 100
cross-gluing station 101
glue line applicator station 102
first polyethylene film unwind station 103
absorbent pad applicator station 104
second polyethylene film unwind station 105
polyethylene printing station 106
tape applicator station 107
heat seal station 108
sheeting station 109

The production process starts with two paper webs 201 and 202 which, in a later stage of the production process, are both cut across their widths so as respectively to provide the paper sheets 5 and 6 of the finished bag assembly 1. As such, paper web 201 has a width X (corresponding to the length of sheet 5) and web 202 has a width Y (corresponding to the length of sheet 6).

Webs 201 and 202 are fed to the printing station 100 where both are printed with the desired information and/or markings (not shown in detail). In the finished bag assembly 2, printed information may be provided on the reverse side of sheet 5 and on the front side of sheet 6. Thus as schematically depicted in Fig 3, the under surface of web 201 and the upper surface of web 202 are printed. However in an alternative arrangement it is possible, say, for the upper surfaces (or the lower surfaces) of the webs 201 and 202 to be printed with one web then being turned through 180° along its length so as to locate the webs in the desired orientation before the subsequent steps of the process.

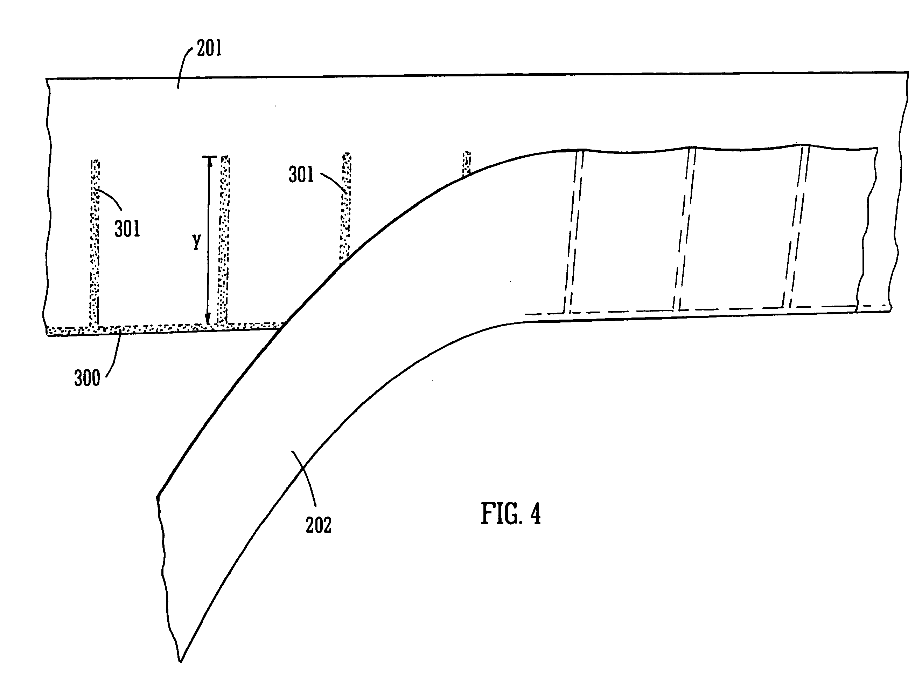

Downstream of the printing station 100, the webs 201 and 202 pass to the cross-gluing station 101 where initially a "ladder-like" glue pattern is continuously laid down on the upper surface of web 201 by means of a servo driven roller 101 a. The ladder-like glue pattern is shown in more detail in Fig 4 and comprises a glue line 300 along one longitudinal edge of web 201 and spaced, transverse glue lines 301. The glue lines 301 have a length of Y (i.e. equal to the width of web 202) and their centre-lines are spaced by a distance corresponding to the width of sheets 6 and 7 in the final bag assembly 1.

Once the ladder-like glue pattern has been applied to web 201, web 202 is laid down onto web 201 and pressed thereon by a roller 101b thereby, in effect, forming pockets along the length of the "assembly" formed by the two webs. For convenience in the subsequent description, this assembly (of webs 201 and 202) is referenced as 203.

Although not illustrated in Fig 3, a line of perforations (for providing the perforations 13 in the final bag assembly 1) may now be formed in the portion of web 201 that projects beyond 202.

A line of glue 204 is now laid onto the exposed upper surface of web 201 by means of a disc 102a at the gluing station 102.

In the next stage of the process a polyethylene web 205 from the first film unwind station 103 is laid down on to the travelling assembly 203. Polyethylene film 205 has a width equal to the length of the polyethylene sheet 9 in the final bag assembly 1 and is laid down on to the assembly 203 so that one longitudinal edge of the polyethylene web 205 is coincident with the edge of assembly 203 to which glue line 300 has been applied and the other longitudinal edge overlies glue line 204. Accurate positioning of the polyethylene web 205 may be achieved by means of a web guide (not shown).

For convenience, the assembly comprised of paper webs 201 and 202 and the polyethylene web 205 is referenced in Fig 3B as 206.

Travelling assembly 206 now passes to the absorbent pad applicator station 104 which is of the "pick-and-place" type. Applicator station 104 is operated such that the absorbent pads 4 are laid down on to the upper surface of polyethylene web 205 with successive pads 4 locating between successive pairs of transverse glue lines 301.

Prior to being laid down on to the polyethylene web 205, the pads 4 are provided with adhesive on their under surface whereby the pads become bonded to the upper surface of polyethylene web 205.

Moving downstream of the absorbent pad application station 104, the travelling web assembly 206 (now with absorbent pads 4 thereon) next reaches the second polyethylene film unwind station 105 from which a polyethylene web 207 is fed to the process. This web 207 has a width corresponding to the length of the polyethylene sheet 10 in the final bag assembly 1. Web 207 is laid down so that its longitudinal edge is coincident with that longitudinal edge of web 206 to which the glue line 300 was applied. Accurate positioning of the polyethylene web 207 may be achieved by means of a web guide (not shown). There is thus a portion of polyethylene web 205 which is not overlaid by the web 207 and it is this portion that forms the flap 12 in the final bag assembly 1.

For convenience, the assembled web is now reference as 208.

On passing through the printing station 106, a printed marking is applied to the upper surface of the polyethylene web 207. The marking may, for example, be a warning such as "Biohazard" or anything else appropriate.

On leaving the printing station 106, the moving web 208 reaches the tape applicator station 107at which a strip of adhesively coated silicon paper 209 is laid adhesive side down over the free edge of polyethylene web 205. This strip 209 provides the combination of paper strip 14 and adhesive 15 in the finished bag assembly 1.

The web assembly now advances from the tape applicator station 107 to the heat seal station 108 where heat sealing is effective in a ladder-like pattern somewhat akin to that shown in Fig 4 so as to bond the polyethylene films 205 and 207 together along their coincident lengthwise edges and along transverse lines which are generally coincident with the transverse glue lines 301 which (ogether with the glue line 300) bond the paper webs 201 and 202 together.

Finally, the web assembly passes to the sheeter station 109 at which the web is cut transversely along the centres of the glue lines 301 (see Fig 4) and thus along the centres of the transverse heat seal lines between the polyethylene webs.

This completes preparation of the form assembly 1 as shown in, and described with reference to, Figs 1 and 2.

It will be appreciated that the process as described above for producing the form assembly 1 involves a number of successive steps which need to be carried out in synchronism with each other to ensure quality of the final form assembly 1.

To this end, a number of control stages may be incorporated in the apparatus and these are described briefly below.

At the printing station 100, each web 201 and 202 may be printed with several colours laid down by respective sets of rotary printing plates (one set for each of webs 201 and 202) with the individual plates of any one set printing one colour so that overall a multi-colour marking may be "built-up" on each of webs 201 and 202. The individual plates of any one set may print a register mark in the form of a dot of the appropriate colour such that, if the final multi-colour pattern on any one web 201 and 202 has been printed correctly, the coloured registration dots are at a predetermined spacing from each other in a line extending along the direction of the running length of the web. A register control system is provided for monitoring the spacing of the printed dots and (*via* a control system not shown) making any necessary adjustments to ensure that the colours print in register. Such control may, for example, be required as rolls holding the webs 201 and 202 to be printed become depleted and therefore of lighter weight with a tendency to be able to run faster. The register control system may, for example, incorporate a camera associated with imaging software for monitoring the relative positions of the dots in each printed set thereof so that any necessary adjustment may be made.

At the printing station 100, the web 201 and/or 202 may be provided with an additional register mark for use in controlling downstream operations of the process.

This additional register mark may be monitored by a respective register control system which is used to control the servo driven roller 101a (at the cross-gluing station 101) to ensure that the glue pattern (shown in Fig 4) is laid down at the appropriate position with regard to the repeating markings on the webs 201 and 202 as printed at the printing station 100.

The same additional register mark may also be used to control operation of the absorbent pad applicator station to ensure that the pads 4.

It will be appreciated that printing station 106, heat seal station 108 and sheater station 109 may also be operated by respective control systems which have regard to the position of the aforementioned additional register mark.

## Claims

1. A bag assembly (1) comprising a backing component and affixed thereto a plastics bag (3) for receiving and holding a container of a liquid sample, the plastics bag (3) being formed from two sheets of liquid impervious plastics material (9, 10) bonded together to form the bag (3) and to leave a mouth therefor and the bag containing a pad of absorbent material (4) **characterised in that** the backing component composes two layers (5, 6) bonded together to form a pouch (2), one of said layers (5) forming the backing component extends beyond the mouth of the pouch (2) to form a flap (8) for the backing component, one of the sheets of plastics material (9) forming the bag extends beyond the mouth of the bag to form a plastics flap (12) for the bag, and the reverse of the plastics flap (12) is bonded to the front of the backing component flap (8) with the plastics bag overlying the mouth of the pouch (2).

2. A bag assembly as claimed in claim 1 wherein the backing component is comprised of paper.

3. A bag assembly as claimed in claim 1 or 2 wherein the pad of absorbent material (4) is bonded to an inner surface of the plastic bag (3).

4. A bag assembly as claimed in any one of claims 1 to 3 wherein the absorbent pad (4) is capable of absorbing 40ml of liquid.

5. A method of producing a bag assembly (1), the method comprising the steps of:
(a) advancing a web (201, 202) of a backing component,
(b) laying onto said advancing web of the backing component an advancing first web (205) of a liquid impervious plastics material and bonding said webs together along a join line (204),
(c) depositing pads (4) of absorbent material at intervals onto the exposed surface of the advancing first plastics web (205),
(d) laying an advancing second web (207) of liquid impervious plastics material onto the first web (205) of plastics material so as to sandwich the absorbent pads (4) between the first and second plastics webs,
(e) bonding said first and second plastics web (205, 207) together along a line extending lengthwise of the plastics webs and along first lines extending transversely of the plastics webs between successive absorbent pads (4), and
(f) severing the web produced in step (e) along said first transverse lines, wherein the backing component comprises first and second webs (201, 202) bonded together along one lengthwise glue line (300) and along spaced second transverse glue lines (301) to form successive pockets in the backing component, wherein, in step (e), the first transverse lines are formed coincident with said second transverse lines (301) and wherein, in step (f), the web is severed along the first and second transverse lines (301), wherein one longitudinal edge of the first web (201) of the backing component is coincident with one longitudinal edge of the second web (202) of the backing component and said coincident longitudinal lines are bonded together along said lengthwise line, wherein the first web (201) of the backing component is wider than the second web (202) thereof whereby said first web has a portion overlapping the second web and wherein the first web of plastics material (205) is, in step (b) affixed along said join line (204) to the overlapping portion on the same face thereof as the second web (202) of the backing component

6. A method as claimed in claim 5 wherein between steps (a) and (b) a line of adhesive (204) is applied to the face of the backing component onto which the first plastics web i(205) s laid and said line of adhesive forms said join line in step (b).

7. A method as claimed in claim 5, wherein the first and second webs of plastics material (205, 207) are laid onto the backing component so as each to have a longitudinal edge coincident with the coincidental longitudinal edges of the first and second webs (201, 202) of the backing component, said coincident longitudinal edges of the first and second webs of plastics material (205, 207) being heat sealed together in step (e).

8. A method as claimed In any one of claims 5 to 7 further comprising the step of:
(a') printing the advancing web of the backing component prior to step (b) of the method.

9. A method as claimed in claim 8 wherein in step (a') a multi-colour print is laid down under register control.

10. A method as claimed in any claim 8 or 9 wherein in step (a') a line of coloured dots are printed and said line of dots is monitored for said register control.

11. A method as claimed in any one of claims 8 to 10 wherein, in step (a') at least one additional register mark is printed and steps (d), (e) and (f) of the method are effected under register control based on said at least one additional register mark.

12. A method as claimed in any one of claims 8 to 10 wherein in step (a') at least one additional register mark is printed, steps (d), (e) and (f) of the method are effective under register control based on said at least one additional register mark, and wherein said second transverse glue lines are laid down with register control in accordance with said one at least additional register mark.

## Patentansprüche

1. Beutelanordnung (1), die eine Stützkomponente aufweist und daran befestigt einen Kunststoffbeutel (3) für das Aufnehmen und Halten eines Behälters mit einer Flüssigkeitsprobe, wobei der Kunststoffbeutel (3) aus zwei Lagen von flüssigkeitsundurchlässigem Kunststoffmaterial (9, 10) gebildet wird, die miteinander verbunden sind, um den Beutel (3) zu bilden, und um eine Öffnung dafür zu lassen, und wobei der Beutel ein Kissen aus saugfähigem Material (4) enthält, **dadurch gekennzeichnet, dass** sich die Stützkomponente aus zwei Schichten (5, 6) zusammensetzt, die miteinander verbunden sind, um eine Tasche (2) zu bilden, wobei sich eine der Schichten (5), die die Stützkomponente bildet, über die Öffnung der Tasche (2) hinaus erstreckt, um eine Lasche (8) für die Stützkomponente zu bilden, wobei sich eine der Lagen des Kunststoffmaterials (9), die den Beutel bildet, über die Öffnung des Beutels hinaus erstreckt, um eine Kunststofflasche (12) für den Beutel zu bilden, und wobei die Rückseite der Kunststofflasche (12) mit der Vorderseite der Stützkomponentenlasche (8) verbunden ist, wobei der Kunststoffbeutel über der Öffnung der Tasche (2) liegt.

2. Beutelanordnung nach Anspruch 1, bei der die Stützkomponente Papier aufweist.

3. Beutelanordnung nach Anspruch 1 oder 2, bei der das Kissen aus saugfähigem Material (4) mit einer Innenfläche des Kunststoffbeutels (3) verbunden ist.

4. Beutelanordnung nach einem der Ansprüche 1 bis 3, bei der das saugfähige Kissen (4) 40 ml Flüssigkeit absorbieren kann.

5. Verfahren zur Herstellung einer Beutelanordnung (1), wobei das Verfahren die folgenden Schritte aufweist:
(a) Vorwärtsbewegen einer Bahn (201, 202) einer Stützkomponente;
(b) Legen einer sich vorwärts bewegenden ersten Bahn (205) eines flüssigkeitsundurchlässigen Kunststoffmaterials auf die sich vorwärts bewegende Bahn der Stützkomponente und Verbinden der Bahnen miteinander längs einer Verbindungslinie (204);
(c) Ablegen der Kissen (4) des saugfähigen Materials in Intervallen auf der freigelegten Fläche der sich vorwärts bewegenden ersten Kunststoffbahn (205);
(d) Legen einer sich vorwärts bewegenden zweiten Bahn (207) des flüssigkeitsundurchlässigen Kunststoffmaterials auf die erste Bahn (205) des Kunststoffmaterials, um so die saugfähigen Kissen (4) zwischen der ersten und der zweiten Kunststoffbahn schichtartig anzuordnen;
(e) Verbinden der ersten und zweiten Kunststoffbahn (205, 207) miteinander längs einer Schicht, die sich in Längsrichtung der Kunststoffbahnen erstreckt und längs erster Schichten, die sich quer von den Kunststoffbahnen zwischen aufeinanderfolgenden saugfähigen Kissen (4) erstrecken; und
(f) Trennen der Bahn, die beim Schritt (e) längs der ersten Querschichten erzeugt wurde, wobei die Stützkomponente eine erste und zweite Bahn (201, 202) aufweist, die entlang einer sich in Längsrichtung erstreckenden Klebstoffschicht (300) und längs beabstandeter zweiter in Querrichtung verlaufender Klebstoffschichten (301) miteinander verbunden werden, um aufeinanderfolgende Vertiefungen in der Stützkomponente zu bilden, wobei beim Schritt (e) die ersten Querschichten mit den zweiten Querschichten (301) übereinstimmend ausgebildet sind, und wobei beim Schritt (f) die Bahn entlang der ersten und zweiten Querschichten (301) getrennt wird, wobei ein Längsrand der ersten Bahn (201) der Stützkomponente mit einem sich in Längsrichtung erstreckenden Rand der zweiten Bahn (202) der Stützkomponente übereinstimmt, und wobei die übereinstimmenden Längsschichten entlang der Längsschicht miteinander verbunden werden, wobei die erste Bahn (201) der Stützkomponente breiter ist als die zweite Bahn (202) davon, wodurch die erste Bahn einen Abschnitt aufweist, der die zweite Bahn überlappt, und wobei die erste Bahn des Kunststoffmaterials (205) beim Schritt (b) entlang der Verbindungslinie (204) am überlappenden Abschnitt auf der gleichen Seite davon wie die zweite Bahn (202) der Stützkomponente befestigt wird.

6. Verfahren nach Anspruch 5, bei dem zwischen den Schritten (a) und (b) eine Klebstoffschicht (204) auf die Seite der Stützkomponente aufgebracht wird, auf die die erste Kunststoffbahn (205) gelegt ist, und die Klebstoffschicht die Verbindungslinie beim Schritt (b) bildet.

7. Verfahren nach Anspruch 5, bei dem die erste und die zweite Bahn des Kunststoffmaterials (205, 207) auf die Stützkomponente gelegt werden, um so jeweils einen Längsrand zu haben, der mit den übereinstimmenden Längsrändern der ersten und zweiten Bahn (201, 202) der Stützkomponente übereinstimmt, wobei die übereinstimmenden Längsränder der ersten und zweiten Bahn des Kunststoffmaterials (205, 207) beim Schritt (e) miteinander heißverschweißt werden.

8. Verfahren nach einem der Ansprüche 5 bis 7, das außerdem den folgenden Schritt aufweist:
(a') Bedrucken der sich vorwärts bewegenden Bahn der Stützkomponente vor dem Schritt (b) des Verfahrens.

9. Verfahren nach Anspruch 8, bei dem beim Schritt (a') ein Mehrfarbendruck unter der Passerkontrolle aufgelegt wird.

10. Verfahren nach einem der Ansprüche 8 oder 9, bei dem beim Schritt (a') eine Linie von farbigen Punkten aufgedruckt und die Linie der Punkte für die Passerkontrolle überwacht wird.

11. Verfahren nach einem der Ansprüche 8 bis 10, bei dem beim Schritt (a') mindestens eine zusätzliche Passermarke gedruckt wird und die Schritte (d), (e) und (f) des Verfahrens unter der Passerkontrolle bewirkt werden, basierend auf der mindestens einen zusätzlichen Passermarke.

12. Verfahren nach einem der Ansprüche 8 bis 10, bei dem beim Schritt (a') mindestens eine zusätzliche Passermarke gedruckt wird, die Schritte (d), (e) und (f) des Verfahrens unter der Passerkontrolle wirksam sind, basierend auf der mindestens einen zusätzlichen Passermarke, und wobei die zweiten quer verlaufenden Klebstoffschichten abgelegt werden, wobei die Passerkontrolle in Übereinstimmung mit der mindestens einen zusätzlichen Passermarke ist.

## Revendications

1. Assemblage de sac (1), comprenant un composant de support et un sac en plastique (3) qui y est fixé, pour recevoir et retenir un récipient d'un échantillon liquide, le sac en plastique (3) étant formé à partir de deux feuilles d'un matériau plastique imperméable aux liquides (9, 10), reliées l'une à l'autre pour former le sac (3) et pour établir une embouchure pour celui-ci, le sac contenant un tampon de matériau absorbant (4), **caractérisé en ce que** le composant de support comprend deux couches (5,6) reliées l'une à l'autre pour former un sachet (2), une desdites couches (5) formant le composant de support s'étendant au-delà de l'embouchure du sachet (2) pour former un rabat (8) pour le composant de support, une des feuilles de matériau plastique (9) formant le sac s'étendant au-delà de l'embouchure du sac pour former un rabat plastique (12) pour le sac, l'envers du rabat en plastique (12) étant relié à l'avant du rabat du composant de support (8), le sac plastique étant superposé à l'embouchure du sachet (2).

2. Assemblage de sac selon la revendication 1, dans lequel le composant de support est composé de papier.

3. Assemblage de sac selon les revendications 1 ou 2, dans lequel le tampon de matériau absorbant (4) est relié à une surface interne du sac en plastique (3).

4. Assemblage de sac selon l'une quelconque des revendications 1 à 3, dans lequel le tampon absorbant (4) est capable d'absorber 40 ml de liquide.

5. Procédé de production d'un assemblage de sac (1), le procédé comprenant les étapes ci-dessous :
(a) déplacement vers l'avant d'une bande (201, 202) d'un composant de support ;
(b) agencement sur ladite bande à déplacement vers l'avant dudit composant de support d'une première bande à déplacement vers l'avant (205) d'un matériau plastique imperméable aux liquides, et liaison desdites bandes le long d'une ligne de liaison (204) ;
(c) dépôt de tampons (4) de matériau absorbant à certains intervalles sur la surface exposée de la première bande plastique à déplacement vers l'avant (205) ;
(d) positionnement d'une deuxième bande à déplacement vers l'avant (207) de matériau plastique imperméable aux liquides sur la première bande (205) de matériau plastique, de sorte à prendre en sandwich les tampons absorbants (4) entre les première et deuxième bandes en plastique ;
(e) liaison desdites première et deuxième bandes en plastique (205, 207) l'une à l'autre le long d'une ligne s'étendant dans le sens de la longueur par rapport aux bandes en plastique et le long de premières lignes s'étendant transversalement par rapport aux bandes en plastique entre des tampons absorbants successifs (4) ; et
(f) séparation de la bande produite lors de l'étape (e) le long desdites premières lignes transversales, le composant de support comprenant des première et deuxième bandes (201, 202) reliées l'une à l'autre le long d'une ligne de collage dans le sens de la longueur (300) et le long de deuxièmes lignes de collage transversales espacées (301), pour former des poches successives dans le composant de support, dans lequel, lors de l'étape (e), les premières lignes transversales sont formées de sorte à coïncider avec lesdites deuxièmes lignes transversales (301), et dans lequel, lors de l'étape (f), la bande est séparée le long des premières et deuxièmes lignes transversales (301), un bord longitudinal de la première bande (201) du composant de support coïncidant avec un bord longitudinal de la deuxième bande (202) du composant de support, lesdites lignes longitudinales coïncidentes étant reliées les unes aux autres le long de ladite ligne dans le sans de la longueur, la première bande (201) du composant de support étant plus large que la deuxième bande (202) de celui-ci, ladite première bande comportant ainsi une partie chevauchant la deuxième bande, la première bande de matériau plastique (205) étant, lors de l'étape (b), fixée le long de ladite ligne de liaison (204) sur la partie à chevauchement sur la même face que la deuxième bande (202) du composant de support.

6. Procédé selon la revendication 5, dans lequel, entre les étapes (a) et (b), une ligne d'adhésif (204) est appliquée sur la face du composant de support sur laquelle est positionnée ladite première bande de plastique (206), ladite ligne d'adhésif formant ladite ligne de liaison lors de l'étape (b).

7. Procédé selon la revendication 5, dans lequel les première et deuxième bandes de matériau plastique (205, 207) sont positionnées sur le composant de support de sorte à avoir un bord longitudinal coïncidant avec les bords longitudinaux coïncidents des première et deuxième bandes (201, 202) du composant de support, lesdits bords longitudinaux coïncidents des première et deuxième bandes de matériau plastique (205, 207) étant reliées par thermoscellage lors de l'étape (e).

8. Procédé selon l'une quelconque des revendications 5 à 7, comprenant en outre l'étape ci-dessous :
(a') impression de la bande à déplacement vers l'avant du composant de support avant l'étape (b) du procédé.

9. Procédé selon la revendication 8, dans lequel, lors de l'étape (a') une impression polychrome est appliquée en présence d'un contrôle de repérage.

10. Procédé selon l'une quelconque des revendications 8 ou 9, dans lequel, lors de l'étape (a'), une ligne de points colorés est imprimée, ladite ligne de points étant surveillée sur la base dudit contrôle de repérage.

11. Procédé selon l'une quelconque des revendications 8 à 10, dans lequel, lors de l'étape (a'), au moins un repère additionnel est imprimé, les étapes (d), (e) et (f) du procédé étant effectuées en présence d'un contrôle de repérage, sur la base dudit au moins un repère additionnel.

12. Procédé selon l'une quelconque des revendications 8 à 10, dans lequel, lors de l'étape (a'), au moins un repère additionnel est imprimé, les étapes (d), (e) et (f) du procédé étant effectuées en présence d'un contrôle de repérage basé sur ledit au moins un repère additionnel, lesdites deuxièmes lignes de collage transversales étant appliquées en présence d'un contrôle de repérage sur la base dudit au moins un repère additionnel.
